# EUROPEAN PATENT APPLICATION

(11) **EP 4 516 234 A1**
(43) Date of publication of application: **05.03.2025**
(21) Application number: 23194218.6
(22) Date of filing: 30.08.2023
(51) Int. Cl.: A61B 6/06, A61B 6/40, A61B 6/00, A61B 6/50, A61B 6/58

(54) **X-RAY IMAGING WITH STEEPER VIEWING ANGLES**

(71) Applicant: Koninklijke Philips N.V., 5656 AG Eindhoven (NL)
(72) Inventor: DRIES, Johan Juliana, Eindhoven (NL); VAN DER WACHT, Rogier, Eindhoven (NL); VAN APELDOORN, Max, 5656AG Eindhoven (NL)
(74) Representative: Philips Intellectual Property & Standards

(57) **Abstract**

The present invention relates to a medical X-ray imaging. In order to provide X-ray imaging having the option for steeper viewing angles, a medical X-ray imaging device (10) is provided that comprises an X-ray source (12) and an X-ray detector (14). The X-ray source is configured to generate an X-ray beam (16). The X-ray detector is configured to receive radiation of the X-ray beam on a detector surface (18) after passing through an object of interest (20). The device is configured to provide an X-ray beam for imaging that covers an area (24) of the detector that is smaller than the detector surface (18) and that is offset with its area center (28) to a center (30) of the detector surface.

## Description

### FIELD OF THE INVENTION

The present invention relates to a medical X-ray imaging device, to a medical X-ray imaging system and to a method for medical X-ray imaging.

### BACKGROUND OF THE INVENTION

In medical imaging, X-ray imaging is used, for example for imaging a region of the heart. In X-ray imaging, X-ray radiation is provided by an X-ray source towards an X-ray detector while an object is arranged between the source and the detector. Larger detector areas provide an increased field of view of the object and are thus preferred. However, it has been shown that large sizes of the detector may limit the viewing angles in view of steep viewing angles in which the viewing axis has the most deviation from a vertical axis.

### SUMMARY OF THE INVENTION

There may thus be a need to provide X-ray imaging having the option for steeper viewing angles.

The object of the present invention is solved by the subject-matter of the independent claims; further embodiments are incorporated in the dependent claims. It should be noted that the following described aspects of the invention apply also for the medical X-ray imaging device, for the medical X-ray imaging system and for the method for medical X-ray imaging.

According to the present invention, a medical X-ray imaging device is provided. The device comprises an X-ray source and an X-ray detector. The X-ray source is configured to generate an X-ray beam. The X-ray detector is configured to receive radiation of the X-ray beam on a detector surface after passing through an object of interest. The device is configured to provide an X-ray beam for imaging that covers an area of the detector that is smaller than the detector surface and that is offset with its area center to a center of the detector surface.

As an effect, the device allows the usage of steeper patient viewing angles when performing interventional procedures, especially in cardiac applications.

As an example, in multi-purpose rooms, a 17 or 20 inch X-ray detector is provided to perform vascular procedures. During cardiac procedures performed in such a room, the physical size of the detector yields limitations in the use of steep angles which are desired by the physicians for a proper view on the vessels around the heart. However, due to the offset radiation and part-use-only of the detector surface, these limitations are at least partly compensated.

As a further particular advantage, this allows the use of so-called multi-purpose rooms without compromise for cardiologists for example.

According to an example, for offsetting the area center of the X-ray beam to the center of the X-ray detector surface, it is provided at least one of the group of i) a shift of the X-ray source; and ii) a tilt of the X-ray source. The shift and tilt are referring to a relative change compared to a centered alignment of the area center of the X-ray beam and the center of the X-ray detector surface.

According to an example, in a first option, the device is configured to provide the X-ray beam for imaging covering an area of the detector that is less than half the area size of the detector surface. In an additional or alternative second option, the device is configured to provide the X-ray beam for imaging that is offset with its area center to a center of the detector surface by at least a third of the distance between the center of the detector surface and a lateral edge of the detector surface.

According to an example, a support arrangement for movably holding the X-ray source and the X-ray detector in relation to each other is provided. The support arrangement is configured to provide an off-center alignment of the X-ray source and the X-ray detector for imaging such that a center beam hits the detector off-center.

According to an example, the support arrangement comprises a first robot arm for holding the X-ray source and a second robot arm for holding the X-ray detector. A control unit is configured to provide for adjusting the relative spatial arrangement of the X-ray source and the X-ray detector in order to achieve the X-ray beam covering the area of the detector that is smaller than the detector surface and to be offset with its area center to the center of the detector surface.

This provides the effect that limitations due to the shape of a C-arc at the side of the X-ray tube is no longer tampering the surgeons' needs for imaging, e.g. flexible imaging.

According to an example, the support arrangement comprises a C-arm structure with the X-ray source and the X-ray detector mounted to opposing ends of the C-arm. The C-arm structure is provided with an adjusting mechanism for adjusting a relative spatial arrangement of the X-ray source and the X-ray detector in order to achieve the X-ray beam covering the area of the detector that is smaller than the detector surface and that offset with its area center to the center of the detector surface.

According to an example, an anti-scatter grid arrangement is provided that is configured for limiting the amount of scattered radiation reaching the X-ray detector. The anti-scatter grid arrangement is configured to be aligned to a respective offset of a center line of the X-ray beam.

According to an example, a collimator arrangement is provided that is configured for limiting the size and shape of the X-ray beam generated by the X-ray source. The collimator arrangement is configured to provide an off-center limitation of the beam in relation to a central axis of the X-ray beam generated by the X-ray source.

In an example, a combination of the anti-scatter grid arrangement and the collimator arrangement is provided.

In an option, the anti-scatter grid arrangement and/or the collimator arrangement is provided for the robot-arm solution.

In another option, the anti-scatter grid arrangement and/or the collimator arrangement is provided for the C-arm solution.

According to the present invention, also a medical X-ray imaging system is provided. The system comprises the medical X-ray imaging device according to one of the preceding examples and an object support. The object support is configured to be arranged between the X-ray source and the X-ray detector during imaging of the object. The medical imaging device is configured to provide image X-ray imaging of the object with stepper viewing angle in with a smaller and offset X-ray beam as compared to X-ray imaging with a full-size and detector-center aligned X-ray beam.

According to an example, the object support is an object table, and the X-ray source and the X-ray detector are configured to provide a steep viewing angle of the object on the object table. According to the present invention also a method for medical X-ray imaging is provided. The method comprises the following steps:
- Arranging an object between an X-ray source and an X-ray detector;
- Generating an X-ray beam by the X-ray source towards the X-ray detector; and
- Detecting X-ray radiation with the X-ray detector.

The generated X-ray beam covers an area of the detector that is smaller than the detector surface and that is offset with its area center to a center of the detector surface.

According to an aspect, an off-center radiation of the detector is provided. As an example, two separate robot arms provide the off-center alignment. According to another aspect, an ability to readout the X-ray detector in an a-centric manner is provided. This allows for a significant increase in projection angle. For good image-quality and reduced amount of X-ray for the patient, the flat detector is positioned as close as possible to the patient.

In an example, two separate motorized robot arms manipulate the detector and the X-ray source separately. In another example, a C-arc based system provides an a-centric movement of the X-ray tube and detector with respect to each other and an a-centric readout of the detector.

As a field of use, all image guided therapies using fixed X-ray systems can be mentioned. In particular, the benefit is high for cardiac interventions in mixed rooms, since these may have a large detector, but also for neuro applications where the solution allows to reach desired projection angles. The present solution is also suitable for neuro applications that work with biplane systems where the combination of two systems itself already limits projection freedom. The offset approach provides advantages here as well.

As an example, for normal X-ray projections, the X-ray source, e.g. an X-ray tube, and the X-ray detector rotate around an iso-center while keeping them aligned such that the middle of the X-ray beam coincides with the center-point of the imaging area of the detector. X-ray images are acquired centric around this center-point and have an adjustable size based on the size of the anatomy the physician is interested in.

The physical size of the detector limits the obtainable projection angles in areas where the detector reaches the chin or shoulders of the patient. However, obtaining larger projection angles in these cases is possible due to the off-center alignment and detector readout and allows: to adjust the position and angle of the X-ray tube, as if a larger projection is possible; to adjust the angle of the detector such that the X-ray beams will coincide with their normal angle of incidence, i.e. parallel to the grid plates in an X-ray grid; and to use an a-centric readout mode of the detector.

The increase in projection angles that can be obtained is definitely relevant for the physicians as it allows them to use projections that can also be obtained with smaller (cardiac) detectors.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1a schematically shows an example of a medical X-ray imaging device; as an option, the medical X-ray imaging device is shown in the context of a medical X-ray imaging system.
Fig. 1b shows another example of a medical X-ray imaging device.
Fig. 2 shows an example of a medical X-ray imaging system in a medical intervention room.
Fig. 3a-3g show illustrations during a movement for achieving an offset setup for X-ray imaging.
Fig. 4 shows basic steps of an example of a method for medical X-ray imaging.

### DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of', when preceding a list of elements, modify the entire list of elements and do not modify the individual elements of the list.

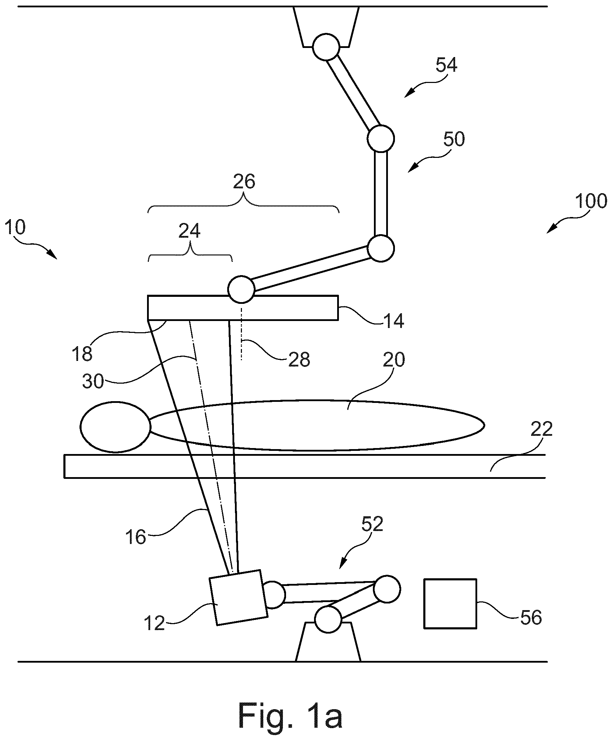

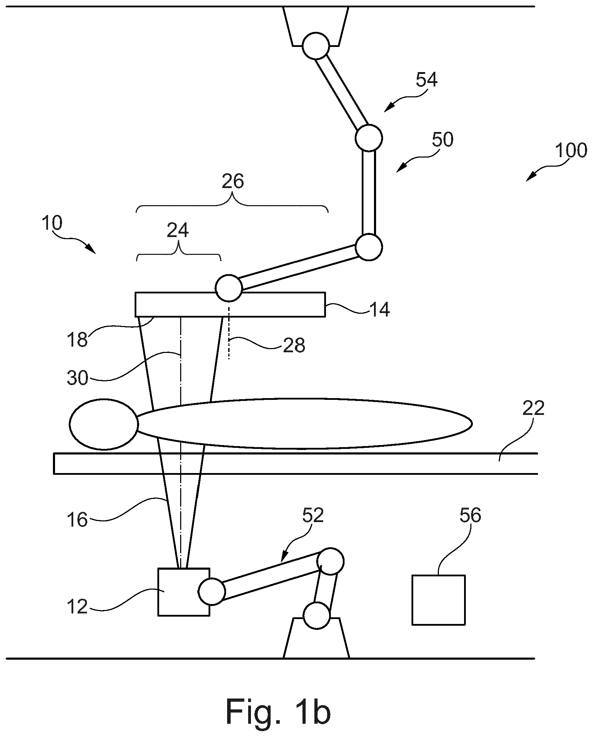

Fig. 1a and Fig. 1b schematically show an example of a medical X-ray imaging device 10. The medical X-ray imaging device 10 comprises an X-ray source 12 and an X-ray detector 14. The X-ray source 12 is configured to generate an X-ray beam 16. The X-ray beam 16 is indicated with two fan-shaped lines. The X-ray detector 14 is configured to receive radiation of the X-ray beam 16 on a detector surface 18 after passing through an object of interest. In Fig. 1a, as an option, the object of interest is indicated as a subject 20 arranged on a subject support 22 like a patient table. The device 10 is configured to provide an X-ray beam for imaging that covers an area 24 of the detector 14 that is smaller than a detector surface area 26 and that is offset with its area center 30 to a center 28 of the detector surface 18. In Fig. 1a and 1b, the area 24 covered by the X-ray beam and the detector surface are indicated with 1D linear brackets, however they refer to 2D areas.

According to an option, for offsetting the area center of the X-ray beam to the center of the X-ray detector surface, it is provided at least one of the group of i) a shift of the X-ray source; and ii) a tilt of the X-ray source. The shift and tilt are referring to a relative change compared to a centered alignment of the area center of the X-ray beam and the center of the X-ray detector surface.

In Fig. 1a, as a first example, the offset is provided as tilting motion. In an example, the X-ray tube 12 is tilted to the left in the drawing.

In Fig. 1b, as a second example, the offset is provided as shifting motion. In an example, the X-ray tube 12 is shifted to the left in the drawing.

As another example, the offset is provided as a combination of a tilting and a shifting motion.

The modified X-ray beam 16 covers only a part of the detector surface 18.

The device 10 is configured to provide a modified X-ray beam 16 impacting on the detector 14. The impacting can also be referred to as impinging or hitting or radiating the detector surface 18 .

Even though steeper viewing angles are provided, 17-inch or 20-inch detectors can still be used, for example, when a larger size is required for peripheral vascular, oncology and other procedures. Despite the larger physical size of these detectors, the present invention allows to reach steep projections which are sometimes required during cardiology procedures, because a collision with the patient's body, e.g. the shoulder area, is prevented due to the off-centered radiation of the detector. Despite the larger physical size of these detectors, the present invention allows to reach steep projections addressing limitations that may be posed by the physical size of the tabletop, for example, when the rotation angle is larger, which implies that the detector is more towards the left or right side of the patient.

The term "detector surface" relates the surface of the detector 14 that contributes for imaging, i.e. the detector surface 18 comprises the effective detector elements that provide image data.

The term "center of the detector surface" relates the geometric midpoint of the detector 14. When using the full, i.e. complete detector surface 18 for standard imaging, the center of the X-ray beam is aligned with the center of the detector surface 18.

In an example, the device 10 comprises an adjustable (e.g. temporarily adjustable) mount of the X-ray source 12 to provide the off-centering of the X-ray beam.

In another example, the device 10 comprises an adjustable (e.g. temporarily adjustable) mount of the X-ray detector 14 to provide the off-centering of the X-ray beam.

In an example, the device 10 comprises adjustable X-ray beam forming means, e.g. a collimator arrangement, to provide the off-centering of the X-ray beam.

As a first option, it is provided that the device 10 is configured to provide the X-ray beam for imaging covering an area of the detector that is less than half the area size of the detector surface 18. As a second option, in addition or alternatively, it is provided that the device 10 is configured to provide the X-ray beam for imaging that is offset with its area center to a center of the detector surface 18 by at least a third of the distance between the center of the detector surface and a lateral edge of the detector surface 18.

In another option, the imaging device 10 is configured to provide an X-ray beam for imaging that is covering an area of the detector 14 that is about a 25-50% smaller in its area size than the area size of the detector surface 18. For example, the device 10 is configured to provide an X-ray beam for imaging that is covering an area of the detector that is about a 30-40% smaller in its area size than the area size of the detector surface 18.

In a further option, the imaging device 10 is configured to provide an X-ray beam for imaging that is covering an area of the detector 14 that is about a 50% smaller in its area size than the area size of the detector surface 18.

In another option, the imaging device 10 is configured to provide an X-ray beam for imaging that is covering an area of the detector 14 that is about a 50-75% smaller in its area size than the area size of the detector surface 18. For example, the device 10 is configured to provide an X-ray beam for imaging that is covering an area of the detector that is about a 60-70% smaller in its area size than the area size of the detector surface 18.

In an example, the imaging device 10 is configured to provide the X-ray beam for imaging covering an area of the detector 14 that is having less than a third or less than a quarter of the size of the detector surface 18.

In an example, the imaging device 10 is configured to provide the X-ray beam for imaging that is offset with its area center to a center of the detector surface 18 by half of the distance between the center of the detector surface and a lateral edge of the detector surface.

In an example, the imaging device 10 is configured to provide the X-ray beam for imaging covering an outer quarter of the detector surface 18. In an example, the outer quarter is arranged along the lateral edge in one of the corners of the detector. In another example, the outer quarter is arranged along the lateral edge between the two corner points.

In Fig. 1a and Fig. 1b, it is shown as an option that imaging device 10 further comprises a support arrangement 50 for movably holding the X-ray source 12 and the X-ray detector 14 in relation to each other. The support arrangement 50 is configured to provide an off-center alignment of the X-ray source 12 and the X-ray detector 14 for imaging such that a center beam hits the detector off-center.

In an example, the support arrangement 50 is capable to provide the off-center alignment.

In an option, the off-center alignment is provided by linear movements (see Fig. 1b). In another option, the off-center alignment is provided by tilting movements (see Fig. 1a). In a further option, the off-center alignment is provided by a combination of linear and tilting movements.

In Fig. 1a and Fig. 1b, the support arrangement 50 comprises a first robot arm 52 for holding the X-ray source 12 and a second robot arm 54 for holding the X-ray detector 14. A control 56 unit is configured to provide for adjusting the relative spatial arrangement of the X-ray source 12 and the X-ray detector 14 in order to achieve the X-ray beam covering the area of the detector 14 that is smaller than the detector surface 18 and that offset with its area center to the center of the detector surface 18.

Such arrangement can also be referred to as C-arm-less, or C-less setup since a C-arm is no longer provided.

The flexibility of such C-less gantry, in which the movements of the detector and X-ray tube are decoupled, in combination with an a-centric (or off-center) readout of the detector 14, overcomes the limitations mentioned above and allows the desired steep angles for imaging.

In an example, the two robot arms 52, 54 are mounted to base structures that are movable in relation to the room. For example, the base structures are mounted to linear tracks (not shown in Fig. 1a and Fig. 1b) arranged aligned to a longitudinal orientation of the object support, such as an object support table or bed.

In another option, not shown in detail, the support arrangement comprises a C-arm structure with the X-ray source 12 and the X-ray detector 14 mounted to opposing ends of the C-arm. The C-arm structure is provided with an adjusting mechanism for adjusting a relative spatial arrangement of the X-ray source and the X-ray detector in order to achieve the X-ray beam covering the area of the detector that is smaller than the detector surface and that is offset with its area center to the center of the detector surface.

In an example, the adjusting mechanism provides for at least two tilt directions of the X-ray tube. As an example, 3x3 preset tilt positions are achievable.

For example, the adjusting mechanism comprises at least two actuators for two different tilting directions.

As an example, the adjusting mechanism comprises a movable mount for the X-ray source and/or the X-ray detector.

As another example, the adjusting mechanism comprises a movable portion between the two opposing arms of the C-arm structure to provide the relative motion between the X-ray source and the X-ray detector.

In a further option, not shown in detail, the imaging device 10 further comprises an anti-scatter grid arrangement configured for limiting the amount of scattered radiation reaching the X-ray detector. The anti-scatter grid arrangement is configured to be aligned to an offset of a center line of the X-ray beam.

In a first option, the anti-scatter grid arrangement is also suitable for a tilted X-ray source position and the grid is aligned with the tilted center beam direction. As an example, the anti-scatter grid arrangement remains in its position. In an example, the X-ray source is tilted such that the point of rotation for the tilting movement is aligned with the focal center point of the X-ray source in a non-offset state.

In a second option, the anti-scatter grid arrangement is also suitable for a shifted X-ray source position and the grid is aligned with the tilted center beam direction. As an example, the anti-scatter grid arrangement is shifted as well. In an example, the X-ray source is shifted such that the anti-scatter grid arrangement is shifted accordingly. The center line of the X-ray beam remains aligned with a center of the anti-scatter grid arrangement.

The anti-scatter grid arrangement is configured to take care that the radiation goes through an anti-scatter grid in the right direction.

In an example, the anti-scatter grid arrangement is tilted.

In another example, the anti-scatter grid arrangement is axially or laterally shifted. In a further example, the anti-scatter grid arrangement is tilted and shifted.

The anti-scatter grid arrangement is offset in such a way that the right position of the focal spot is maintained, or re-arranged.

In a still further option, not shown in detail, the imaging device 10 further comprises a collimator arrangement configured for limiting the size and shape of the X-ray beam generated by the X-ray source. The collimator arrangement is configured to provide an off-center limitation of the beam in relation to a central axis of the X-ray beam generated by the X-ray source.

The collimator arrangement can be provided as a shutter or size-adaption arrangement.

The collimator arrangement can also be referred to as off-middle collimator.

As an option, the detector 14 is provided with a read-out unit that is configured to provide a part-readout of the detector surface relating to the area that is covered by the X-ray beam.

As another option, the detector 14 is provided with a read-out unit that is configured to provide a complete readout of the detector; detector data from areas not covered by the offset X-ray beam are dismissed during image data processing.

The so-called black parts of the detector are deleted after readout.

As indicated above, as an option, the medical X-ray imaging device 10 is shown in the context of a medical X-ray imaging system 100 in Fig. 1a and Fig. 1b.

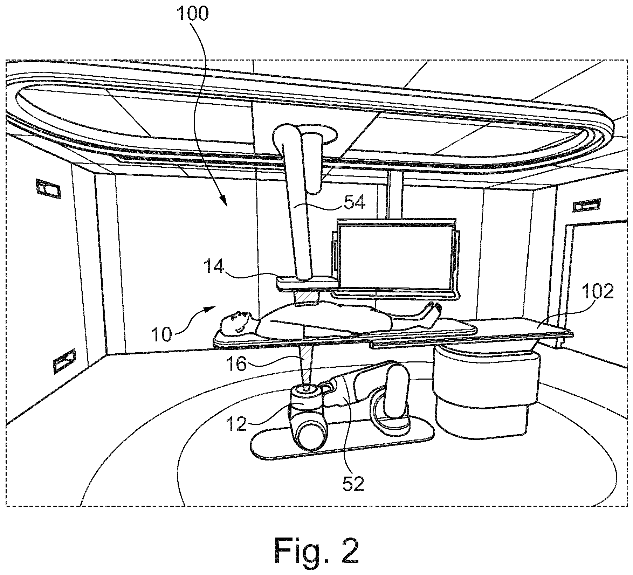

Fig. 2 shows an example of a medical X-ray imaging system 100 in a medical intervention room. The medical X-ray imaging system 100 comprises the medical X-ray imaging device 10 according to one of the preceding examples and options and an object support 102. The object support 102 is configured to be arranged between the X-ray source 12 and the X-ray detector 14 during imaging of the object. The medical imaging device 100 is configured to provide image X-ray imaging of the object with stepper viewing angle and offset X-ray beam as compared to X-ray imaging with a full-size and detector-center aligned X-ray beam.

As an option, the object support 102 is an object table and the X-ray source 12 and the X-ray detector 14 are configured to provide a steep viewing angle of the object on the object table.

As an option, a viewing angle in the range of approximately 30° to 5° is provided. The viewing angle is referring to an angle between the center of the modified X-ray beam and an axis of a subject support like a patient table.

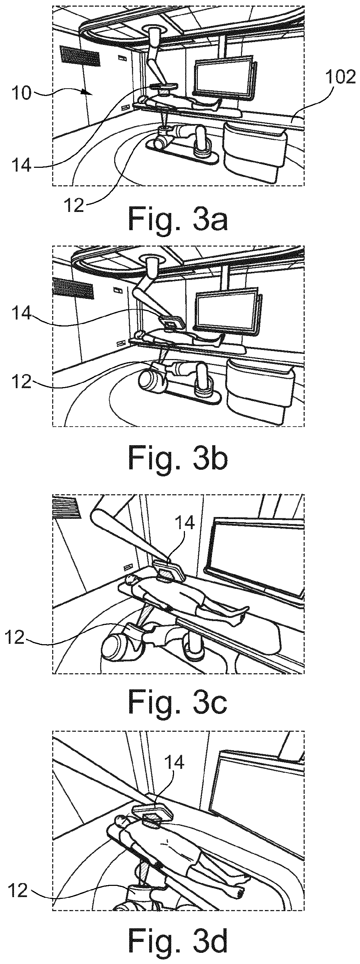

Fig. 3a-3g show illustrations during a movement for achieving an offset setup for X-ray imaging. It is noted that the X-ray beam is indicated throughout the illustrations for an easier understanding of the geometrical setup. However, the X-ray beam is only provided during imaging and not during the positioning procedure.

Fig. 3a shows an arrangement with a vertical viewing angle with X-ray beam and detector surface aligned in a centered way.

Fig. 3b shows an arrangement with an inclined viewing angle with the X-ray beam and the detector surface aligned in a centered way. The viewing angle is transverse to a longitudinal axis of the subject support, i.e. transverse to the subject. The X-ray source 12 and the X-ray detector 14 are commonly rotated in their spatial position.

Fig. 3c shows an arrangement with a further inclined viewing angle with the X-ray beam being still centered on the detector surface. The X-ray source 12 and the X-ray detector 14 are further rotated in their spatial position.

Fig. 3d shows an arrangement with the viewing angle also inclined to the subject support and the subject. The X-ray beam are still centered on the detector surface. The X-ray source 12 and the X-ray detector 14 are commonly rotated in their spatial position.

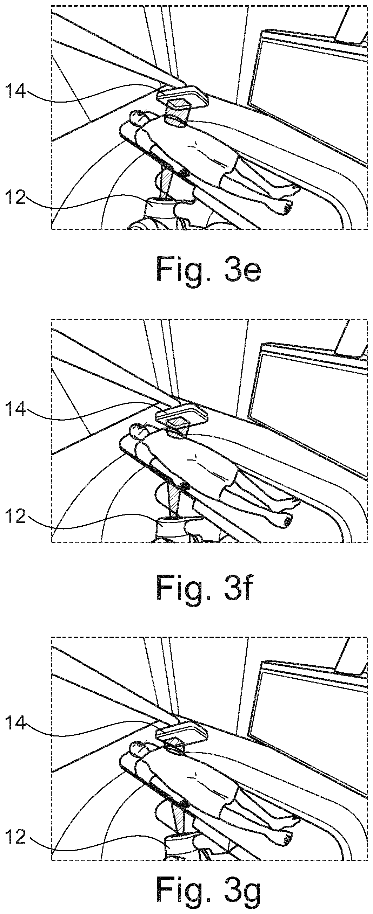

Fig. 3e shows an arrangement with the X-ray beam moved towards the left lower edge of the detector 14. As an example, the X-ray source 12 is adapted in its orientation. The positioning stage shown in Fig. 3e may be obtained by moving and rotating the detector with respect to the X-ray beam. This results in more space being created towards the patient's body and/or a tabletop edge. As an example, the X-ray source 12 and the X-ray detector 14 are commonly rotated in their spatial position.

Fig. 3f shows an arrangement with an even more increased inclination of the viewing angle. The X-ray source 12 and the X-ray detector 14 are rotated in their spatial position.

Fig. 3g shows an arrangement with the viewing angle in a maximum inclination, i.e. the steepest viewing angle. The X-ray source 12 and the X-ray detector 14 are again rotated in their spatial position.

In this position, the user can then perform the actual imaging by generating a respectively off-centered X-ray beam.

As can be seen, the approach of the present invention to off-center the X-ray beam allows a steeper viewing angle despite a rather large detector. The steeper viewing angle increases the viewing options for a surgeon or the like. An increase in flexibility is provided.

Of course, also other moving options are provided in order to find a steep viewing angle as in Fig. 3g.

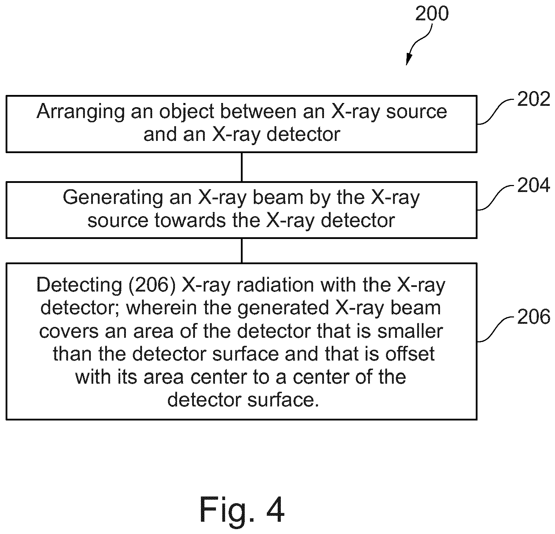

Fig. 4 shows basic steps of an example of a method 100 for medical X-ray imaging. The method 200 comprises the following steps: In a first step 202, an object is arranged between an X-ray source and an X-ray detector. In a second step 204, an X-ray beam is generated by the X-ray source towards the X-ray detector. In a third step 206, X-ray radiation is detected with the X-ray detector. The generated X-ray beam covers an area of the detector that is smaller than the detector surface and that is offset with its area center to a center of the detector surface.

In an option, a computer program is provided that comprises instructions which, when the program is executed by a computer, cause the computer to carry out the method 100.

In an example, a computer program or program element for controlling an apparatus according to one of the examples above is provided, which program or program element, when being executed by a processing unit, is adapted to perform the method steps of one of the method examples above.

In an example, a computer readable medium having stored the computer program of the example above is provided.

The term "subject" may also be referred to as individual. The "subject" may further also be referred to as patient, although it is noted that this term does not indicate whether any illness or disease is actually present with the subject.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit or be distributed over more than one computer units, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

Aspects of the invention may be implemented in a computer program product, which may be a collection of computer program instructions stored on a computer readable storage device which may be executed by a computer. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors.

As discussed above, the processing unit, for instance a controller implements the control method. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

This exemplary embodiment of the invention covers both, a computer program that right from the beginning uses the invention and a computer program that by means of an update turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A medical X-ray imaging device (10) comprising:
- an X-ray source (12); and
- an X-ray detector (14);
wherein the X-ray source is configured to generate an X-ray beam;
wherein the X-ray detector is configured to receive radiation of the X-ray beam on a detector surface after passing through an object of interest;
wherein the device is configured to provide an X-ray beam for imaging that covers an area of the detector that is smaller than the detector surface and that is offset with its area center to a center of the detector surface.

2. Device according to claim 1, wherein, for offsetting the area center of the X-ray beam to the center of the X-ray detector surface, it is provided at least one of the group of:
i) a shift of the X-ray source; and
ii) a tilt of the X-ray source.

3. Device according to claim 1 or 2, wherein the device is configured to provide the X-ray beam for imaging:
i) covering an area of the detector that is less than half the area size of the detector surface; and/or
ii) that is offset with its area center to a center of the detector surface by at least a third of the distance between the center of the detector surface and a lateral edge of the detector surface.

4. Device according to claim 1, 2 or 3, further comprising a support arrangement (50) for movably holding the X-ray source and the X-ray detector in relation to each other;
wherein the support arrangement is configured to provide an off-center alignment of the X-ray source and the X-ray detector for imaging such that a center beam hits the detector off-center.

5. Device according to claim 4, wherein the support arrangement comprises a first robot arm (52) for holding the X-ray source and a second robot arm (54) for holding the X-ray detector; and
wherein a control unit (56) is configured to provide for adjusting the relative spatial arrangement of the X-ray source and the X-ray detector in order to achieve the X-ray beam covering the area of the detector that is smaller than the detector surface and that offset with its area center to the center of the detector surface.

6. Device according to claim 4, wherein the support arrangement comprises a C-arm structure with the X-ray source and the X-ray detector mounted to opposing ends of the C-arm; and
wherein the C-arm structure is provided with an adjusting mechanism for adjusting a relative spatial arrangement of the X-ray source and the X-ray detector in order to achieve the X-ray beam covering the area of the detector that is smaller than the detector surface and that offset with its area center to the center of the detector surface.

7. Device according to one of the preceding claims, further comprising an anti-scatter grid arrangement configured for limiting the amount of scattered radiation reaching the X-ray detector;
wherein the anti-scatter grid arrangement is configured to be aligned to an offset of a center line of the X-ray beam.

8. Device according to one of the preceding claims, further comprising a collimator arrangement configured for limiting the size and shape of the X-ray beam generated by the X-ray source;
wherein the collimator arrangement is configured to provide an off-center limitation of the beam in relation to a central axis of the X-ray beam generated by the X-ray source.

9. Device according to one of the preceding claims, wherein the detector is provided with a read-out unit that is configured to provide a part-readout of the detector surface relating to the areas that is covered by the X-ray beam.

10. Device according to one of the preceding claims, wherein the detector is provided with a read-out unit that is configured to provide a complete readout of the detector and wherein detector data from areas not covered by the offset X-ray beam are dismissed during image data processing.

11. A medical X-ray imaging system (100) comprising:
- the medical X-ray imaging device (10) according to one of the preceding claims; and
- an object support (102);
wherein the object support is configured to be arranged between the X-ray source and the X-ray detector during imaging of the object; and
wherein the medical imaging device is configured to provide image X-ray imaging of the object with stepper viewing angle in with a smaller and offset X-ray beam as compared to X-ray imaging with a full-size and detector-center aligned X-ray beam.

12. System according to claim 11, wherein the object support is an object table and wherein the X-ray source and the X-ray detector are configured to provide a steep viewing angle of the object on the object table.

13. A method (200) for medical X-ray imaging comprising the following steps:
- arranging (202) an object between an X-ray source and an X-ray detector;
- generating (204) an X-ray beam by the X-ray source towards the X-ray detector; and
- detecting (206) X-ray radiation with the X-ray detector;
wherein the generated X-ray beam covers an area of the detector that is smaller than the detector surface and that is offset with its area center to a center of the detector surface.

14. Computer program comprising instructions which, when the program is executed by a computer, cause the computer to carry out the method of claim 13.
